# EUROPÄISCHE PATENTANMELDUNG

(11) **EP 3 646 773 A1**
(43) Veröffentlichungstag der Anmeldung: **06.05.2020**
(21) Anmeldenummer: 18000866.6
(22) Anmeldetag: 05.11.2018
(51) Int. Cl.: A61B 1/12, A61B 1/00, A61B 90/70

(54) **VERFAHREN ZUR REINIGUNG, AUFBEREITUNG UND PRÜFUNG VON HOHLKÖRPERINSTRUMENTEN, INSBESONDERE ENDOSKOPEN**

(71) Anmelder: VSZ GmbH Versorgungs- und Servicezentrum für Medizinischen Bedarf GmbH, 3500 Krems (AT)
(72) Erfinder: Weiß, Franz, 3034 Maria Anzbach (AT); Reither, Ulrike, 3130 Herzogenburg (AT)

(57) **Zusammenfassung**

Die Erfindung betrifft ein Verfahren zur Reinigung und Desinfektion von medizinischen Hohlkörperinstrumenten, insbesondere (flexiblen) Endoskopen, gekennzeichnet durch folgende Verfahrensschritte:
a) Konditionierung des medizinischen Hohlkörperinstruments beispielsweise des Endoskops unmittelbar nach dem Ende des mit dem Instrument durchgeführten Eingriffs
b) Entfernen der Ventile und Zuführung der wiederverwendbaren und aufbereitbaren Ventile gemeinsam mit dem Instrument zu einer Transportbox
c) Transportieren der Transportbox mit den darin verwahrten Ventilen und dem Instrument zu einer Aufbereitungsstelle
d) Zuführen der Ventile und des Endoskops zu einem Reinigungs- und Desinfektionsvorgang innerhalb von 48 Stunden nach Ende des mit dem Instrument durchgeführten Eingriffs
e) ggf. Durchführung einer Vorreinigung durch
f) Zuführung und Anschließen der Instrumente zu/an einem Reinigungs- und Desinfektionsgerät, wobei jeder Kanal getrennt angeschlossen wird und
g) Prüfung der Durchgängigkeit und Dichtigkeit
h) Durchführen der Reinigung im Reinigungs- und Desinfektionsgerät nach den vorgegebenen Parametern
i) ggf. anschließend zusätzliches Trocknen des Instruments in einem Trockenschrank.

## Beschreibung

Die Erfindung betrifft ein Verfahren zur Reinigung, Aufbereitung und Prüfung von Hohlkörperinstrumenten, insbesondere Endoskopen, bei dem durch die vorgesehenen Verfahrensschritte eine schonende, validierbare und sichere Aufbereitung von keimfreien Endoskopen erfolgt, wodurch die Nutzdauer der Instrumente deutlich verlängert wird.

Aus EP 0 603 563 A1 ist ein Verfahren zur Prüfung und Reinigung von Endoskopen mit einem Kopfteil und einem Einführungsschlauch sowie ein Reinigungsgerät zur Durchführung dieses Verfahrens bekannt.
Die Reinigung umfasst mindestens einen Reinigungsdurchgang, während dessen wenigstens der Einführungsschlauch mindestens äußerlich einer unter Druck stehenden Reinigungsflüssigkeit ausgesetzt wird, wobei vor dem ersten Reinigungsdurchgang eine Prüfung des Endoskops durchgeführt wird, bei welcher über einen Druckprüfanschluss Druckgas in das Innere des Endoskops geleitet und mindestens der Innendruck des Endoskops daraufhin überprüft wird, ob er innerhalb eines Prüfintervalls einen bestimmten Prüfdruck erreicht und der Reinigungsdurchgang nur bei positivem Resultat der Prüfung ausgelöst wird und dabei dann im Endoskop ein über dem Druck der Reinigungsflüssigkeit liegender Innendruck aufrechterhalten wird.

Aus EP 1 105 061 B1 ist ein Verfahren zum Reinigen eines verunreinigten Lumens eines Endoskops bekannt, bei dem ein Wischglied, das einen Durchmesser hat, der geringer ist als der Durchmesser des Lumens und ein vollständig umlaufendes hinteres Wischelement aufweist in Axialrichtung durch das Lumen des Endoskops gezogen oder geschoben wird. Dabei wird die Innenwand des Lumens abgewischt, sodass Verunreinigungen, die nach dem Durchgang des Wischelements zurückbleiben, gleichförmig als dünner Film auf der Innenwand des Lumens verteilt sind. Dieser dünne Film wird anschließend mit einer Reinigungszusammensetzung behandelt.

Aus US 2016/0030984 A1 ist ein Reinigungsschiffchen zur Reinigung von medizinischen Hohlkörperinstrumenten bekannt, das durch magnetische Anziehung durch einen Kanal zu einer Beförderungsvorrichtung geführt wird, die gleitend außerhalb des Kanals gelagert ist. Dadurch soll ein Steckenbleiben des Reinigungsschiffchens im Kanal verhindert werden.

Aus EP 1 153 568 B1 und EP 2 927 829 A2 sind Reinigungsmanagementsysteme bekannt, bei denen der Reinigungsprozess von medizinischen Instrumenten, wie Endoskopen geplant und überwacht werden kann.

Die aus dem Stand der Technik bekannten Verfahren konzentrieren sich bei der Reinigung und Aufbereitung medizinischer Instrumente auf einzelne Prozesse bzw. auf einzelne Parameter, die in den in diesen Prozessen eingehalten bzw. erfüllt werden müssen, wie zum Beispiel Durchflussmenge pro Zeiteinheit, Druck bzw. Druckveränderungen, zu verwendende Reinigungsflüssigkeiten oder Aufbereitungschemikalien, deren Konzentrationen und dergleichen, die Schritte vor bzw. nach diesen Prozessen werden allerdings völlig außer Acht gelassen.

Unter einem medizinischen Hohlkörperinstrument werden im Folgenden insbesondere (flexible) Endoskope verstanden, die bei minimalinvasiven Eingriffen an Menschen oder Tieren zur Anwendung kommen. Beispiele für derartige medizinische Hohlkörperinstrumente sind Bronchoskope, Uteroskope, Cytoskope, Ureterorenoskope, Gastroskope, Coloskope, Dudenoskope und dergleichen.

Aufgabe der vorliegenden Erfindung ist es, ein Verfahren zur Reinigung bzw. Desinfektion, Aufbereitung und Prüfung von Hohlkörperinstrumenten, insbesondere Endoskopen bereitzustellen, das den gesamten Aufbereitungsprozess zum Erhalt eines keimfreien desinfizierten Endoskops umfasst und die Reihenfolge und zeitliche Abstimmung der Maßnahmen berücksichtigt.

Gegenstand der Erfindung ist daher ein Verfahren zur Reinigung und Desinfektion von medizinischen Hohlkörperinstrumenten, insbesondere (flexiblen) Endoskopen, gekennzeichnet durch folgende Verfahrensschritte:
a) Konditionierung des medizinischen Hohlkörperinstruments beispielsweise des Endoskops unmittelbar nach dem Ende des mit dem Instrument durchgeführten Eingriffs durch
   -) Reinigung der äußeren Oberfläche des Einführschlauches durch Abwischen mit einem Einwegtuch ohne Verwendung eines Desinfektionsmittels oder eines Reinigungsverstärkers,
   -) Prüfung der Kanäle des Einführschlauchs und des Instruments auf Durchgängigkeit durch Spülen mit vollentsalztem Wasser bis klares vollentsalztes Wasser austritt,
   -) Füllen der Kanäle mit Reinigungsflüssigkeit
b) Entfernen der Ventile und Zuführung der wiederverwendbaren und aufbereitbaren Ventile gemeinsam mit dem Instrument zu einer Transportbox
c) Transportieren der Transportbox mit den darin verwahrten Ventilen und dem Instrument zu einer Aufbereitungsstelle
d) Zuführen der Ventile und des Endoskops zu einem automatisierten Reinigungs- und Desinfektionsvorgang innerhalb von 48 Stunden nach Ende des mit dem Instrument durchgeführten Eingriffs
e) ggf. Durchführung einer Vorreinigung durch
   -) Durchziehen eines Reinigungswerkzeugs, durch die Kanäle des Instruments, insbesondere durch den Versorgungs- und Biopsiekanal zur Überprüfung auf im Kanal verbliebene Rückstände
   -) Vorreinigung des Ventilsitzes und Schaftes mit einer Einmalbürste,
   -) Kontrolle der mechanischen Teile, insbesondere der Hebel,
   -) Überprüfung der Beweglichkeit der Spitze, die im OP- Gebiet eingesetzt wird
   -) aus- und durchblasen des Dichtigkeitstesters mit medizinischer Druckluft
f) Zuführung und Anschließen der Instrumente zu/an einem Reinigungs- und Desinfektionsgerät, wobei jeder Kanal getrennt angeschlossen wird und
g) Prüfung der Durchgängigkeit und Dichtigkeit,
h) Durchführen der Reinigung im Reinigungs- und Desinfektionsgerät nach den vorgegebenen Parametern
i) ggf. anschließend zusätzliches Trocknen des Instruments in einem Trockenschrank.

Der Trocknungsschritt i) kann ggf. entfallen, falls das Instrument für die sofortige Verwendung vorgesehen ist.
Sofern das Instrument erst für spätere Verwendung vorgesehen ist, wird durch den zusätzlichen Trocknungsschritt die Anwendungsmöglichkeit des Instruments auf bis zu 6 Monaten verlängert.

Im ersten Schritt wird jedes medizinische Gerät konditioniert. Unmittelbar nach jedem Gebrauch (in der Regel sofort nach Ende des mit dem Instrument durchgeführten Eingriffs) wird der Einführschlauch des Endoskops ohne Verwendung von Desinfektionsmittel oder Reinigungsverstärker beispielsweise mit einem Einwegtuch äußerlich abgewischt.
Es folgt die Prüfung der Kanäle des Instruments auf Durchgängigkeit durch Spülen am Turm mit vollentsalztem-Wasser (3-5 µS) bis klares Wasser austritt. Anschließend werden die Kanäle des Instruments mit einer Reinigungslösung gefüllt. Zweckmäßigerweise erfolgt die Befüllung aus vorbereiteten Messbechern .

Besonders bewährt hat sich als Reinigungslösung das im Handel erhältliche WL-Clean IC, da dieses Mittel ausgezeichnet kriechwirksam ist und Fett und/oder sonstige Rückstände unterwandert, und weder anorganische noch organische Rückstände in den Kanälen hinterlässt. Es sind aber auch alle anderen handelsüblichen Reinigungslösungen für medizinische Geräte geeignet.

Anschließend werden die Ventile des Instruments entfernt: Einwegventile werden fachgerecht entsorgt, wiederaufbereitbare Ventile werden zum Endoskop in eine desinfizierte gesicherte Transportbox gelegt. Geeignet sind insbesondere Transportboxen, die innen mit Noppenmatten ausgelegt sind.

Der Transport innerhalb und/oder außerhalb des Krankenhauses (beispielsweise zur Aufbereitungsstelle und zurück) erfolgt in speziellen Boxen und Fahrzeugen, die rein/unrein/ be/entladen und nach Gebrauch mit einem validiertem Verfahren desinfizierend gereinigt werden, um die Verbreitung von Krankheitserregern durch Kontakt mit Mitarbeitern, Flüssigkeiten, Aerosolen, und dgl. zu verhindern. Derartige Verfahren sind bekannt und beispielsweise in CN 2014-69708U offenbart.

Die Hohlkörperinstrumente müssen nun innerhalb von 48 Stunden nach dem Ende des Eingriffs der endgültigen Reinigung zugeführt werden.
Für ein gesichertes Gesamtergebnis ist zudem auch eine Rückverfolgbarkeit für die Gesamtzeit von maximal 48 Stunden bis zur eigentlichen Aufbereitung, sowie der zeitliche Ablauf generell (Eingriffsende, Abteilung, Person die die Konditionierung durchgeführt hat, Uhrzeit der Konditionierung, etc. die händisch an den Begleitpapieren vermerkt oder elektronisch mittels Barcodezuordnung zum Transportbehälter dokumentiert werden) einzuhalten.
Sämtliche Transportwege und Übergabezeiten werden ebenfalls dokumentiert.

Die Reinigung erfolgt unter Einhaltung der gesetzlichen Sicherheitsbestimmungen. Die medizinischen Hohlkörperinstrumente werden durch das Durchziehen eines Reinigungswerkzeugs, beispielweise eine Sweepers, oder dergleichen, durch die Kanäle des Instruments, wie z.B. durch den Versorgungs- und Biopsiekanal auf eine korrekte Konditionierung geprüft. Ein entsprechendes Verfahren ist beispielsweise in EP 1 105 061 B offenbart.

Danach werden die Endoskope inspiziert und personifiziert, und ggf. vorgereinigt. Es erfolgt eine zweckmäßigerweise händische Vorreinigung des Ventilsitzes und Schaftes mit einer Einmalbürste, die Kontrolle der Hebel, Überprüfung der Beweglichkeit der Spitze, aus/durchblasen des Dichtigkeitstesters mit Druckluft. Dabei wird ausschließlich medizinische Druckluft mindestens der Klasse 3 nach ISO 8573 verwendet.
Diese Prüfungen erfolgen jeweils nach den vorgeschriebenen Arbeitsanweisungen und werden nach einer Checkliste abgearbeitet.

Abschließend werden die Kanäle des Instruments an die Verbindungsstecker eines zugeordneten Reinigungs- und Desinfektionsgeräts geräte- und endoskopspezifisch angeschlossen und die Seriennummer des Endoskops eingescannt.

Die Durchgängigkeitsprüfung und Dichtigkeitsprüfung werden pro Kanal, pro Endoskop durchgeführt, registriert und mit den Anfangsdaten im Neuzustand verglichen Ein derartiges Verfahren ist beispielsweise aus DE102005014429 B3 bekannt.

Unterschiede zwischen den Daten im Neuzustand und im Zustand nach Verwendung und Reinigung geben Aufschluss über Veränderungen im Instrument gegenüber dem Neuzustand beispielweise ob Einengungen, Blockaden, oder Undichtigkeiten vorhanden sind.

Nur wenn die erreichten Werte des Einzelkanaldrucks (also der Druck jedes einzelnen Kanals des Instruments) innerhalb der vorgegebenen Grenzen sind, wird das Endoskop nach dem Prozessablauf maschinell freigegeben. Derartige Verfahren ist beispielsweise in CA2616348A1 oder US20020161460A1 beschrieben.

Der erste Reinigungsschritt wird ohne Reinigungsmittel nur mit vollentsalztem Wasser (3-5 µS), der zweite Reinigungsschritt mit einem Reinigungsmittel und der dritte bzw. jeder weitere Reinigungsschritt ohne Reinigungsmittel (jeweils lediglich mit vollentsalztem Wasser mit 3-5 µS) durchgeführt.

Zwischen den Reinigungsschritten muss das Reinigungswasser gewechselt werden.
Die Trocknungszeit ist verlängert und beträgt im Reinigungs- und Desinfektionsgerät mindestens 12 Minuten.

Die Prozesse in den Reinigungs- und Desinfektionsgeräten werden aufgezeichnet, kontrolliert und freigegeben. Entsprechende Verfahren sind beispielsweise aus EP1153568A2 oder EP2927829A1 bekannt.

Nach der Aufbereitung im Reinigungs- und Desinfektionsgerät erfolgen die Kontrollen am Endoskop analog den Kontrollen vor der Aufbereitung laut Checkliste und die Zusammenführung laut Packliste sowie das Trocknen.

Die Ventile werden mit medizinischer Druckluft (mindestens Klasse 3 nach ISO 8573) trockengeblasen und dem Endoskop zugeordnet.

Für die Außentrocknung werden spezielle, sterile partikelarme Tücher insbesondere Tücher nach EN ÖN 13795 aus 100% Mikrofaser verwendet.
Der validierte (dokumentierte, freigegeben mit festgelegtem Ablaufdatum) Trocknungsprozess für innen erfolgt im Trocknungsschrank nach hinterlegter Trocknungszeit je Endoskop, jedoch mindestens für 20 Minuten.
Das Zubehör wird laut Packliste seinem Endoskop wieder zugefügt.

Ureterorenoskop, Bronchoskop, Cystoskop oder ähnliche medizinische Geräte werden ohne Distalschutz in einer Sterilisationsfolie verpackt, nach einem bekannten validierten Verfahren (H₂O₂, Formaldehyd) sterilisiert und in einer eigenen, verplombten Box ausgeliefert.

Der Rücktransport, intern wie extern, erfolgt wie der Transport direkt nach der Anwendung des Endoskops.

Die Übergabe der Boxen sowohl zum Transport zur Aufbereitungsstelle als auch zum Rücktransport erfolgt in einem versperrbaren Raum, um Verwechslungen ausschließen zu können.

Auf der beigelegten Packliste oder am aufgeklebten Barcode auf der Box sind mindestens folgende Daten aufgelistet:
Endoskopnummer, Zubehör, Datum, Uhrzeit, Person, die die Aufbereitung durchgeführt hat, jeweilige Freigaben. Entsprechende Verfahren sind bekannt und beispielsweise in EP1153568A2 oder EP2927829A1 beschrieben.

Wesentlich ist die Einhaltung aller beschriebenen Verfahrensschritte, insbesondere ist die Füllung der Kanäle mit einer Reinigungslösung unmittelbar nach Ende des durchgeführten Eingriffs, sowie die Einhaltung des Zeitintervalls von maximal 48 Stunden bis zum Reinigungs- und Desinfektionsvorgang essentiell.

Sollte nur ein Schritt nicht durchführbar sein oder nicht das erforderliche Ergebnis erzielen, wird das Aufbereitungsverfahren abgebrochen und das Instrument zur Reparatur gebracht.

Nach einem abgestimmten Gesamtplan werden Prüfungen (biologisch, toxikologisch, etc.) sowie die Validierung des Gesamtprozesses an sich durch eine akkreditierte Prüfstelle durchgeführt.
Darunter fallen insbesondere folgende Schritte:
Einmal jährlich erfolgen Prüfungen der Reinigungs- und Desinfektionsgeräte nach dem Stand von Technik und Wissen (EN ISO 15883-4, 15.6.2016) durch ein akkreditiertes Institut, sowie die qualitative und quantitative mikrobiologische Prüfung aller Endoskope nach der Aufbereitung.
Dies erfolgt durch eine definierte Probenahme (laut Arbeitsanweisung) nach der Aufbereitung, indem ein Arbeits- und Biopsiekanal mit einer 30 ml Lösung (Trypton-NaCl) geprüft und anschließend mit 20 ml Luft nachgespült wird.
Die Spüllösungen werden innerhalb von 24 h in einem akkreditierten Prüfinstitut qualitativ und quantitativ mikrobiologisch untersucht.

Halbjährlich erfolgt die Wartung des Reinigungs- und Desinfektionsgerätes, jedoch spätestens nach 500 Aufbereitungen.

Quartalsweise erfolgt eine Prüfung der Schläuche (2m, 2mm) auf Restproteine, die mit koaguliertem Schafsblut, als Surrogat beim Endoskopaufbereitungsprozess exponiert wurden.
Weiters werden 200 ml Proben vollentsalztes Wasser des letzten Spülgangs in den Reinigungs- und Desinfektionsgeräts qualitativ und quantitativ mikrobiologisch, sowie das letzte Spülwasser biologisch und toxikologisch untersucht.

Monatlich erfolgt eine Prüfung jedes Endoskops durch eine Spülung mit 1% SDS (Natriumdodecylsulfat) Lösung (laut Arbeitsanweisung), die auf den Proteingehalt in einem akkreditiertem Prüfinstitut geprüft wird.

Zweimal wöchentlich werden Reinigungsindikatoren beim ersten Aufbereitungsprozess mitexponiert, beurteilt, bewertet und dokumentiert. Proteinschnelltests werden ebenfalls in diesem Intervall durchgeführt.

## Patentansprüche

1. Verfahren zur Reinigung und Desinfektion von medizinischen Hohlkörperinstrumenten, insbesondere (flexiblen) Endoskopen, **gekennzeichnet durch** folgende Verfahrensschritte:
a) Konditionierung des medizinischen Hohlkörperinstruments beispielsweise des Endoskops unmittelbar nach dem Ende des mit dem Instrument durchgeführten Eingriffs durch
-) Reinigung der äußeren Oberfläche des Einführschlauches durch Abwischen mit einem Einwegtuch ohne Verwendung eines Desinfektionsmittels oder eines Reinigungsverstärkers,
-) Prüfung der Kanäle des Einführschlauchs und des Instruments auf Durchgängigkeit durch Spülen mit vollentsalztem Wasser bis klares vollentsalztes Wasser austritt
-) Füllen der Kanäle mit Reinigungsflüssigkeit
b) Entfernen der Ventile und Zuführung der wiederverwendbaren und aufbereitbaren Ventile gemeinsam mit dem Instrument zu einer Transportbox
c) Transportieren der Transportbox mit den darin verwahrten Ventilen und dem Instrument zu einer Aufbereitungsstelle
d) Zuführen der Ventile und des Endoskops zu einem Reinigungs- und Desinfektionsvorgang innerhalb von 48 Stunden nach Ende des mit dem Instrument durchgeführten Eingriffs
e) ggf. Durchführung der Vorreinigung durch
-) Durchziehen eines Reinigungswerkzeugs durch die Kanäle des Instruments, insbesondere durch den Versorgungs- und Biopsiekanal zur Überprüfung auf im Kanal verbliebene Rückstände
-) Vorreinigung des Ventilsitzes und des Schaftes,
-) Kontrolle der mechanischen Teile, insbesondere der Hebel,
-) Überprüfung der Beweglichkeit der Spitze, die im OP- Gebiet eingesetzt wird,
-) aus- und durchblasen des Dichtigkeitstesters mit medizinischer Druckluft
f) Zuführung und Anschließen der Instrumente zu/an einem Reinigungs- und Desinfektionsgerät, wobei jeder Kanal getrennt angeschlossen wird und
g) Prüfung der Durchgängigkeit und Dichtigkeit
h) Durchführen der Reinigung im Reinigungs- und Desinfektionsgerät nach den vorgegebenen Parametern
i) ggf. anschließend zusätzliches Trocknen des Instruments in einem Trockenschrank.

2. Verfahren nach Anspruch 1, **dadurch gekennzeichnet, dass** die Spülvorgänge in Schritt a) mit vollentsalztem Wasser mit 3 - 5 µS erfolgen.

3. Verfahren nach einem der Ansprüche 1 oder 2, **dadurch gekennzeichnet, dass** als Reinigungswerkzeug in Schritt e) ein Sweeper verwendet wird.

4. Verfahren nach einem der Ansprüche 1 bis 3, **dadurch gekennzeichnet, dass** die Vorreinigung des Ventilsitzes und des Schafts händisch mit einer Einmalbürste erfolgt.

5. Verfahren nach einem der Ansprüche 1 bis 4, **dadurch gekennzeichnet, dass** die Innentrocknung der Instrumente nach dem Reinigungs- und Desinfektionsvorgang durch Durchblasen mit medizinischer Druckluft getrocknet werden.

6. Verfahren nach einem der Ansprüche 1 bis 5, **dadurch gekennzeichnet, dass** als Reinigungsmittel in Schritt a) ein kriechwirksames Reinigungsmittel verwendet wird.

7. Verfahren nach einem der Ansprüche 1 bis 6, **dadurch gekennzeichnet, dass** zwischen den Reinigungsschritten das vollentsalzte Wasser gewechselt wird.

8. Verfahren nach einem der Ansprüche 1 bis 7, **dadurch gekennzeichnet, dass** in Schritt e) und g) medizinische Druckluft mindestens der Klasse 3 nach ISO 8576 verwendet wird.

9. Verfahren nach einem der Ansprüche 1 bis 8, **dadurch gekennzeichnet, dass** die Außentrocknung des Hohlköperinstruments durch partikelarme Tücher nach EN ÖN 13795 erfolgt.

10. Verfahren nach einem der Ansprüche 1 bis 9, **dadurch gekennzeichnet, dass** die Innentrockung der Instrumente durch medizinische Druckluft mindestens der Klasse 3 nach ISO 8576 erfolgt.

11. Verfahren nach einem der Ansprüche 1 bis 10 **dadurch gekennzeichnet, dass** alle Verfahrensschritte überprüft und dokumentiert werden.

12. Verfahren nach einem der Ansprüche 1 bis 11, **dadurch gekennzeichnet, dass** als medizinische Hohlkörperinstrumente flexible Endoskope, wie Bronchoskope, Uteroskope, Cytoskope, Ureterorenoskope, Gastroskope, Coloskope, Dudenoskope und dergleichen den Reinigungsschritten unterworfen werden.

13. Verfahren nach einem der Ansprüche 1 bis 12, **dadurch gekennzeichnet, dass** die Prüfung und Validierung des Verfahrens durch folgende Schritte erfolgt:
-) einmal jährlich: Prüfungen der Reinigungs- und Desinfektionsgeräte nach EN ISO 15883-4, 15.6.2016 durch ein akkreditiertes Institut, sowie die qualitative und quantitative mikrobiologische Prüfung aller Endoskope nach der Aufbereitung,
-) halbjährlich: Wartung des Reinigungs- und Desinfektionsgerätes, jedoch spätestens nach 500 Aufbereitungen,
-) quartalsweise: Prüfung der Schläuche auf Restproteine qualitative und quantitative mikrobiologische, biologische, und toxikologische Untersuchung von Proben des vollentsalzten Wassers des letzten Spülgangs in den Reinigungs- und Desinfektionsgeräten und des letzten Spülwassers,
-) monatlich: Prüfung jedes Endoskops durch eine Spülung mit 1% SDS Lösung, die auf den Proteingehalt in einem akkreditierten Prüfinstitut geprüft wird,
-) zweimal wöchentlich: Mitexponieren, Beurteilen, Bewerten und Dokumentieren von Reinigungsindikatoren beim ersten Aufbereitungsprozess, Durchführung von Proteinschnelltests.
